(19) Europäisches Patentamt
European Patent Office
Office européen des brevets

(11) Publication number: 0 085 866
B1

(12) **EUROPEAN PATENT SPECIFICATION**

(45) Date of publication of patent specification: **09.04.86**

(51) Int. Cl.$^4$: **C 07 D 417/12,** A 61 K 31/54
// C07D275/06

(21) Application number: **83100504.6**

(22) Date of filing: **21.01.83**

(54) **Derivatives of benzo-1,2-thiazine-1,1-dioxide, a process for their preparation and pharmaceutical compositions containing the same.**

(30) Priority: **05.02.82 IT 1948682**

(43) Date of publication of application:
**17.08.83 Bulletin 83/33**

(45) Publication of the grant of the patent:
**09.04.86 Bulletin 86/15**

(84) Designated Contracting States:
**AT DE FR GB IT NL SE**

(56) References cited:
**US-A-4 309 427**

(73) Proprietor: **CHIESI FARMACEUTICI S.p.A.**
**Via Palermo, 30**
**I-43100 Parma (IT)**

(72) Inventor: **Chiesi, Paolo**
**Via Palermo 30**
**I-43100 Parma (IT)**
Inventor: **Villani, Flavio**
**Via Palermo 30**
**I-43100 Parma (IT)**

(74) Representative: **Bianchetti, Giuseppe et al**
**Studio Consulenza Brevettuale Via Rossini, 8**
**I-20122 Milan (IT)**

EP 0 085 866 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention refers to new derivatives of benzo-1,2-thiaxine-1,1-dioxide of general formula:

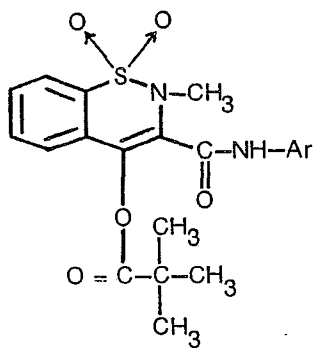

(I)

wherein Ar stands for 2-pyridyl or 2-thiazolyl and their addition salts with pharmaceutically acceptable acids.

The compounds included in the formula (I) are

4-pivaloyloxy-2-methyl-2H-1,2-benzothiazine-3-[N-(2-pyridyl)-carboxamide]-1,1-dioxide; 4-pivaloyloxy-2-methyl-2H-1,2-benzothiazine-3-[N-(2-thiazolyl)-carboxamide]-1,1-dioxide.

In the US—A—3,591,584 a new class of compounds of the series of the 3-aminocarbonyl-1,2-benzothiazine-1,1-dioxide bearing heterocyclic residues at the amino-nitrogen has been reported for the first time.

Compounds of this kind are endowed with an analgesic and antiinflammatory activity, and some of them have been used in the therapy of arthrorheumatic affections, in spite of presenting drawbacks especially due to the appearance of gastroenteric disorders in the treated patients; disorders, however, very common in other non-steroidic antiinflammatory compounds.

US—A—4,309,427 discloses acyl derivatives of N-(2-pyridyl)-2-methyl-4-hydroxy-2H-1,2-benzothiazine-6-carboxamide-1,1-dioxide having antiinflammatory activities.

After our priority-filing of the present patent application (Italian Application n. 19486 A/82 of February 5, 1982) we have learned from the publication of the European Application n. 57059, filed on January 11, 1982, that also some esters of 4-hydroxy-2-methyl-2H-1,2-benzothiazine-3-[N-(2-pyridyl)-carboxamide]-1,1-dioxide have been prepared and pharmacologically tested.

These new derivatives, nevertheless, do not present any real therapeutical advantage, since they possess, to a lower or a higher extent, the same characteristics as the starting compound; they simply turn out to be more suitable to the topic administration route.

It has instead been found, and it is the object of the present invention, that the esterification of the hydroxyl in position 4 in the structure in question with a pivaloyl substituent allows to obtain a new compound with antiinflammatory activity that presents unforeseeable surprising advantages at therapeutic level.

It has in fact been established that only this particular new derivative corresponding to the formula (I), precisely obtained by means of esterification of the hydroxyl in 4-position with a tert-butyl radical, while it keeps substantially unaltered the antiinflammatory activity, it has a drastically reduced ulcerogenic activity, thus showing a therapeutic index 10.25 times higher than the one of the starting compound.

Various ways of synthesis followed to obtain the compounds described in the above-mentioned patents and other similar compounds are known from the prior art. One of these ways can be schematized as follows:

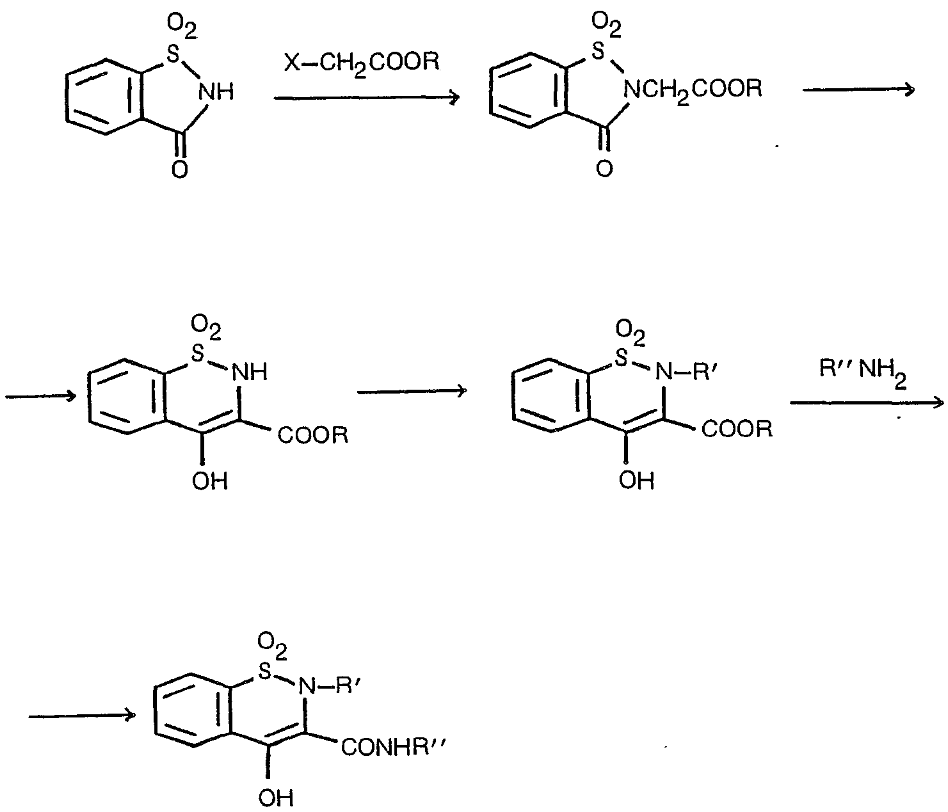


(wherein R and R′ represent for example $CH_3$, whereas R′′ represents for example H, alkyl, heteroaromatic residue); see US—A—3,591,584, 1971; Lombardino J. G. et al., J. Med. Chem. 14 (12), 1171, 1971.

Other alternative ways of synthesis described in patents and in the literature have not offered up to now particular advantages as compared to one indicated above.

It has now been found, and it is the object of the present invention, a new method to prepare the compounds of formula (I), which presents some interesting advantages.

According to the invention, the compounds (I) are obtained in conformity to the following reaction scheme, where Ar has the meaning above specified for the formula (I), whereas X and $X_1$ are halogens, for example Cl or Br.

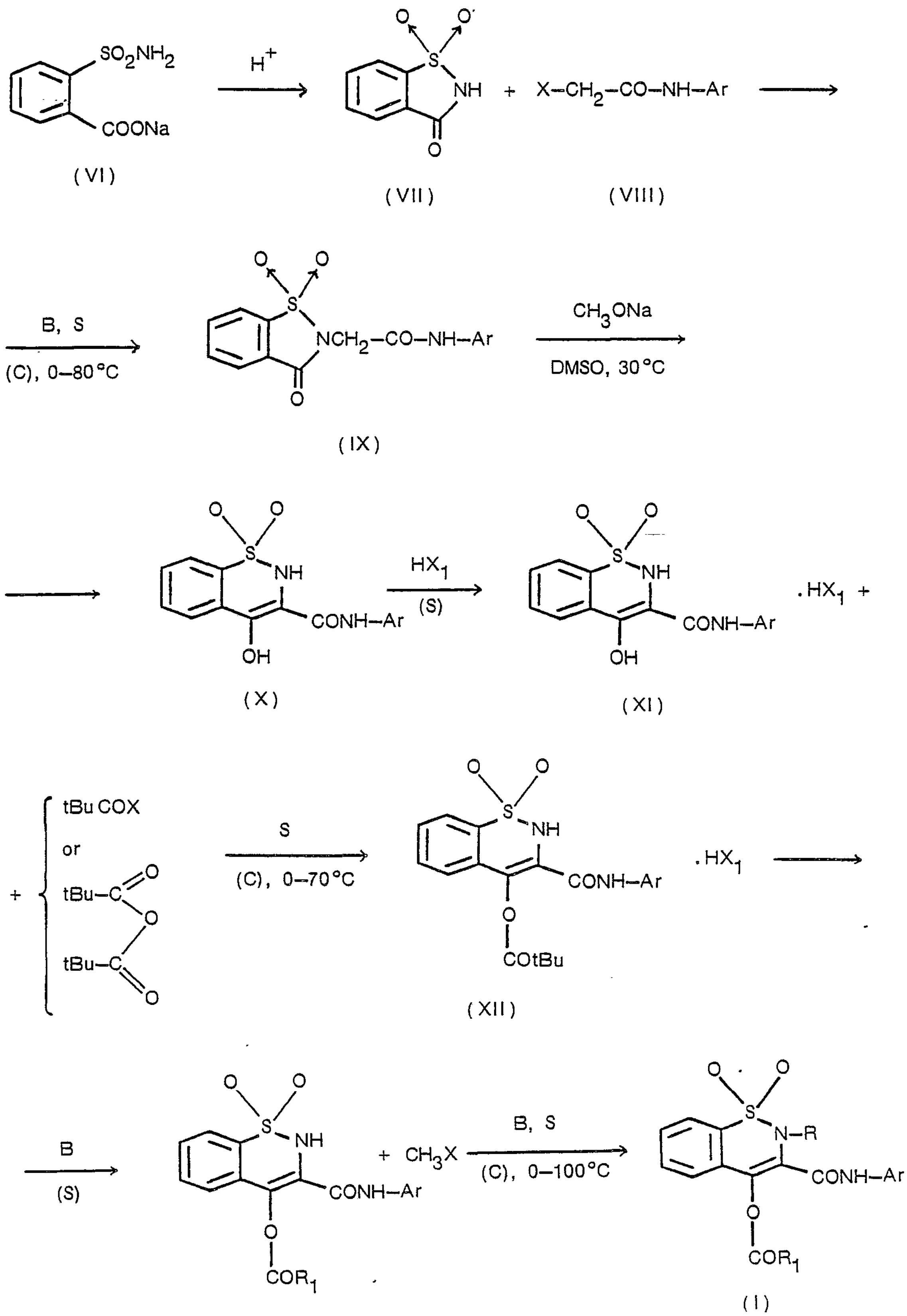


(VI) (VII) (VIII) (IX) (X) (XI) (XII) (XIII) (I)

As it is evident, the main characteristic of the new process of synthesis of the invention is the fact of obtaining, from a pentaatomic derivative, an esaatomic ring already substituted in 3-position with the N-heterocyclic carboxamidic group. This involves a remarkable increase of the yields.

The general synthesis scheme is afterwards explained in a more detailed way.

The starting products (VI) and (VII) are commercially available or they can be prepared according to well-known methods.

As a matter of fact the compound (VII) can be obtained by neutralizing exactly with mineral acids the sodium salt (VI) dissolved in water, filtering, if necessary, the unreacted product, acidifying at pH 1—0 and filtering VII.

The intermediate VII is subsequently reacted with the compound X—$CH_2$CONHAr (VIII). This reagent is often commercially available, or its synthesis is well-known or it is easily obtainable according to well-known methods.

The reaction between the intermediate (VII) and the product (VIII) is carried out at temperatures ranging from 0 and 80°C, preferably from room temperature to the boiling temperature of the solvent that is used, and, if necessary, in the presence of a stoichiometric or catalytic quantity of a base and/or of an acidity acceptor (B), such as aliphatic, aromatic or heterocyclic tertiary amines, hydroxides or carbonates of alkali or earth-alkali metals, bicarbonate, acetates, hydrides, amides or alcoxides of alkali metals.

As solvents (S) all the ones that are commonly used, such as benzene, toluene, acetone, chloroform, 1,2-dichloro-ethane, dimethylformamide, water, methylene chloride, etc. can be used. It is also possible to carry out the reaction in double phase using water and one of the abovementioned solvents. In some cases the solvent can be represented by the reagent itself used in excess.

The reaction may be carried out in presence of a catalyst (C), such as, for instance, a base like the ones described under "B", quaternary ammonium or phosphonium salts (especially in the double phase reactions), alkali iodides, or cuprous iodide, etc.

The expansion reaction of the thiazolic ring takes place by means of treatment of the intermediate (IX) with sodium methoxide in dimethylsulfoxide and by checking the exothermia of the reaction so that the temperature is not higher than 30—35°C.

The intermediate (X) thus obtained is transformed at a temperature that is lower than 5°C in one of its addition salts with mineral acids ($HX_1$) (preferably HCl), by addition of the acid (or of one of its solutions in solvent) to a solution of the intermediate (X) in a solvent that allows the precipitation of the previously formed salt. The intermediate (XI) is reacted with pivaloyl halide (tBu COX) or with the corresponding anhydride of a carboxylic acid [(tBU $CO)_2O$] in a solvent that can be chosen among the ones described before (S) (with the exception of water) and at temperatures ranging from 0 to 70°C. Also a catalyst (C) can be used, but more often this is superfluous. When the reaction is over the intermediate (XII) is isolated, and it is immediately transformed in the compound (XIII) by means of treatment with a stoichiometric quantity of a base (B) and/or in the presence of a solvent (S).

The intermediate (XIII) is finally reacted with methyl halide Mex in the presence of bases "B" in the absence or in the presence of solvents "S" and of catalysts "C", at temperatures ranging from 0 to 100°C, preferably at room temperature. For this reaction it is possible to use, as solvents, lower alcohols, from which it is possible to crystallize the final product (I).

The product of formula (I) can be prepared alternatively starting from the intermediate (X), by means of the following reactions:

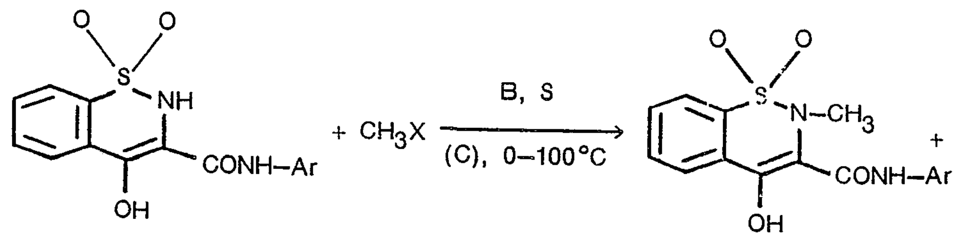


(X) (XIV)

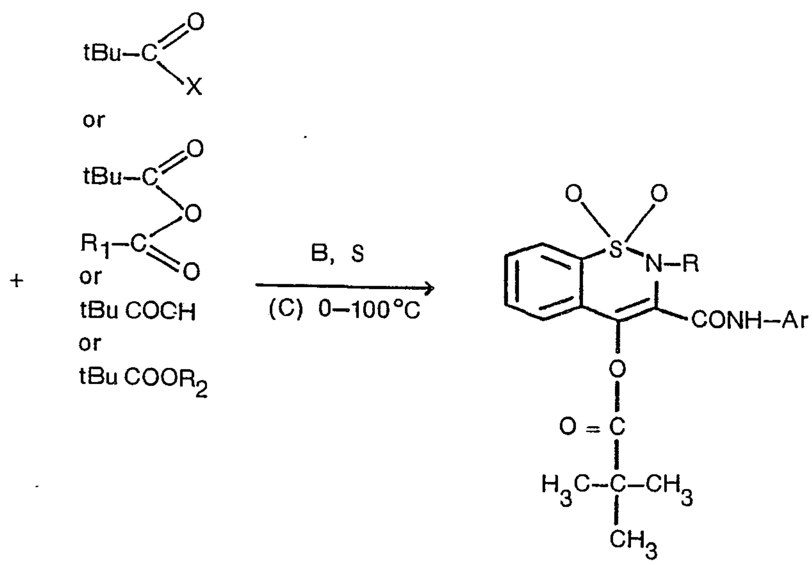


(I)

where Ar has the meanings that have been previously defined for the general formula (I); $R_2$ stands for a lower alkyl; X stands for a halogen such as Cl, Br;

B, S, C have the meanings indicated above.

The intermediate (X) is reacted with Me in the presence of a base B, in the absence or in the presence of the solvent S and of the catalyst C, at temperatures ranging from 0 to 100°C, preferably at room temperature. The intermediate (XIV) that has been thus obtained is reacted with pivaloyl halides, pivalic acid or pivalic acid anhydride or esters. The base B, the solvent S and the catalyst C are of the above-mentioned kind. Also acid catalysts are suitable (for example mineral acids such as sulphuric, phosphoric and hydrochloric acid). The reaction is carried out between 0—100°C, more often between room temperature and 60°C or at the boiling temperature of the solvent.

The final product of the general formula (I) is crystallized from solvents such as alcohols, ethers, methylene chloride, acetone etc., or from their mixtures.

The compounds of formula (I) thus obtained can moreover form addition salts with various pharmaceutically acceptable acids such as inorganic acids like hydrochloric acid, sulphuric acid, phosphoric acid, nitric acid, hydrobromic acid, or organic acids such as oxalic acid, maleic acid, fumaric acid, malic acid, tartaric acid, citric acid, ascorbic acid.

Those salts can be easily prepared in a well-known way, for example by adding an equimolecular quantity or an excess of the acid to a solution of the compound of formula (I) in an organic solvent, such as methanol, ethanol, isopropanol, acetone and similar. The invention is explained in a more detailed way by the following examples, which, on the other hand, do not limit it.

## Example 1

a) Preparation of intermediate (VII)

In a 500 ml beaker provided with magnetic stirrer 0.06 moles of intermediate VI, which are dissolved in about 200 ml of $H_2O$, are charged. When the dissolution is complete, 1N hydrochloric acid is slowly added to exact neutrality (checking by means of indicator or pH-meter). The reaction mixture is now left under stirring for about 30′ and the solid formed, if any, is filtered off.

The filtrate is acidified to pH 1—0.

The precipitated VII is filtered, washed with $H_2O$ and dried in oven under vacuum. Yields vary from 65 to 90%. The product obtained is characterized by means of N.M.R., I.R., U.V. spectra and of elementary analysis.

b) Preparation of the intermediate IX

In a 1-liter flash with stirrer, thermometer, reflux condenser, dropping funnel, external thermosthatic bath, 0.25 moles of intermediate (VII), 23 g of potassium carbonate, 200 ml of $H_2O$ are charged. The reaction mixture is heated to 50°C for 25 minutes, then 8.5 g of tetrabutyl ammonium hydrogen sulphate are charged, thereafter the reaction mixture is stirred again for 18′ at 50°C and 200 ml of benzene are charged and, under fast stirring, a solution of 0.3 moles of intermediate (VIII) dissolved in 250 ml of benzene, are slowly dropped in about 20′. The mixture is then refluxed for 70′, cooled at room temperature, the phases are separated and the organic phase is washed with slightly acid $H_2O$, then with 1N KOH and finally with water to neutrality. The benzenic phase is dried on $K_2CO_3$, thereafter the solvent is evaporated under reduced pressure. A crude product is obtained in a yield of 60—80% [intermediate IX]. The product is characterized, after crystallization from suitable solvents (for instance lower alcohols), by means of N.M.R., I.R. spectra and of elementary analysis.

c) Preparation of the intermediate XI

In a 500 ml flask provided with stirrer, thermometer, dropping funnel and external cooling bath 100 ml of dimethylsulphoxide (anhydrous), 0.2 moles of intermediate (IX) are charged and then, under fast stirring, a thick suspension of 21.5 g of sodium methoxide suspended in 100 ml of anhydrous dimethylsulphoxide is added in about one hour and in such a way as to not exceed 30°C of internal temperature.

When the addition is over, the reaction mixture is stirred again for 20′ then acidified with 3N HCl and extracted with $CHCl_3$. After drying, the $CHCl_3$ phase is evaporated, the residue [intermediate (X)] is dissolved in ethanol and transformed into the hydrochloride by addition of ethanolic HCl at 0°C. The hydrochloride [intermediate (XI)] is obtained in a yield of 36—67% and it often does not call for crystallization. It can be characterized by means of its N.M.R. I.R. spectra and of elementary analysis.

d) Preparation of the intermediate (XIII)

In a 500 ml flask provided with stirrer, reflux condenser, external thermostatic bath 0.15 moles of intermediate (XI), 150 ml of 1,2-dichloroethane (anhydrous) are charged, the reaction mixture is stirred for 15′ at room temperature, then cooled to 2—3°C and 0.16 moles of acyl chloride $R_1COCl$ are charged in one portion.

The reaction mixture is thereafter stirred for 30′ at 0—5°C allowing then the temperature to raise in about 90′ to 20°C, and then heated in about 60′ to 60—70°C. During this heating there is development of HCl continuing again for about 3 hours. At the end the reaction mixture is allowed to cool to room temperature and the product obtained is filtered [intermediate (XII)]. The intermediate (XII) is suspended in about 200 ml of $H_2O$ and the suspension is then brought to pH = 7.3 with 10% KOH, keeping the temperature cool and under fast stirring. The suspension is stirred for about two hours and then the product obtained is filtered [intermediate (XIII)] which can be re-crystallized from isopropanol and characterized by means of N.M.R. I.R. spectra and of elementary analysis. Yields are 78—92%.

e) Preparation of compound (I)

In a 1000 ml reactor provided with stirrer and thermometer, dropping funnel, 0.12 moles of intermediate (XIII), 400 ml of ethanol, 100 ml of water are charged. The reaction mixture is stirred for 15′, then 120 ml of 1N NaOH are added and the mixture is again stirred for 20′ at room temperature; 0.38 moles of the alkyl halide RX are then charged, dropping in about 25′. The reaction is allowed to proceed for 18 hours at room temperature, the precipitate formed is filtered, washed with $H_2O$ and finally dried. Yields are 65—90%. The product is characterized by means of N.M.R. I.R. spectra and of elementary analysis.

Example 2

a) Preparation for the intermediate (XIV) from the intermediate (X)

The reaction is carried out in completely analogous conditions to those described in point e) of Example 1.

Yields are 45—80%.

The product obtained having the formula XIV is characterized by means of N.M.R. I.R. spectra and of elementary analysis.

b) Preparation of final compound I

In a 1000 ml flask provided with stirrer, thermometer, reflux condenser, dropping funnel and external thermostatic bath, 0.133 moles of the intermediate (XIV), 300 ml of anhydrous 1,2-dichloroethane and 6.9 g of triethylamine are charged, the mixture is then heated to 50°C and a solution of acyl halide $R_1COX$ (0.099 moles) diluted in 15 ml of dichloroethane is dropped (20′). A slight exothermia is noticed during the addition, at the end of which stirring is continued for 20′ at 50°C. 6.8 g of triethylamine are then charged and

stirring is continued again for 15′ at 50°C, thereafter a solution of the acyl halide $R_1COX$ (0.099 moles) in 15 ml of 1,2-dichloroethane is dropped in about 20′.

At the end of this second dropping, the temperature is still kept for 20′ at 50°C, the reaction mixture is then left to cool at 30°C and filtered in order to remove the solid formed (triethylamine hydrochloride).

The filtrate is washed with 2 × 200 ml of $H_2O$, then with 2 × 200 ml of 0.5N NaOH and finally with $H_2O$ to neutrality. The solution in 1,2-dichloroethane is dried on $Na_2SO_4$, the solvent is then evaporated under vacuum till the obtaining of an oil which immediately solidifies and which is well pulped in ethyl ether. The suspension is filtered, washed with ethyl ether and the solid obtained, which does not require recrystallization, is dried. Yields are 75—90%. The products are characterized by means of N.M.R. I.R. spectra and of elementary analysis. With the above described methods, the following products are obtained:

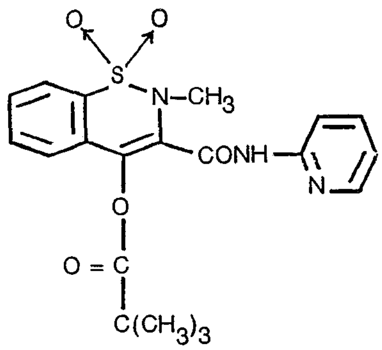

XV

4-Pivaloyloxy-2-methyl-2H-1,2-benzothiazine-3-[N-(2-pyridyl)-carboxamide]-1,1-dioxide.

White crystalline powder melting at 152—154°C.

$^1H$*—NMR Spectrum* at 60 MHz ($CDCl_3$) (ppm, δ) = 9.0 (s, broad, mobile —NH—); 8.35—8.15 (m, 2H = H-5 and H-6 of the pyridine ring); 8.0—7.4 [m, 5H (4 aromatics + H-3 of the pyridine ring)]; 7.3—6.9 (m, 1H = H-4 of the pyridine ring); 3.05 (s, 3H = —N—$CH_3$); 1.4 [s, 9H = —$CH_3)_3$].

*I.R. Spectrum* (KBr) 3700—3150 $cm^{-1}$ (broad); 2980 $cm^{-1}$ (weak); 1760 $cm^{-1}$; 1680 $cm^{-1}$; 1530 $cm^{-1}$; 1435 $cm^{-1}$; 1350 $cm^{-1}$; 1300 $cm^{-1}$; 1090 $cm^{-1}$; 765 $cm^{-1}$.

*Elementary Analysis* (C, H, N) for $C_{20}H_{21}N_3O_5S$

calcd. % C = 57.82; H = 5.095; N = 10.11
found % C = 57.69; H = 5.087; N = 10.28.

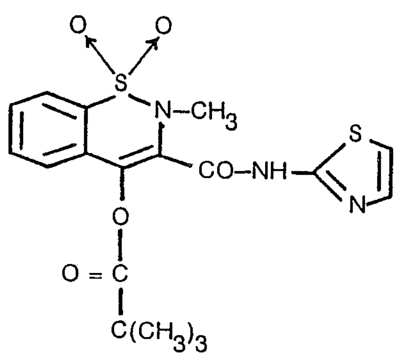

(XVI)

4-Pivaloyloxy-2-methyl-2H-1,2-benzothiazine-3-[N-(2-thiazolyl)carboxamide]-1,1-dioxide.

White cream powder melting at 175—177°C.

$^1H$*—NMR Spectrum* at 60 MHz ($CDCl_3$) (ppm, δ) = 11.1—9.9 (s, broad 1H mobile —NH—); 8.0—7.55 (m, 4H, aromatics); 7.5 and 7.0 (2 doublets of 1H each, J ≃ 4Hz = 2h thiazolics); 3.0 (s, 3H = N—$CH_3$); 1.4 (s, 9H = —$C(CH_3)_3$).

*I.R. Spectrum* (KBr) 3680—3220 $cm^{-1}$ (broad); 2980 $cm^{-1}$ (weak) 1760 $cm^{-1}$; 1675 $cm^{-1}$; 1560 $cm^{-1}$; 1530 $cm^{-1}$; 1350 $cm^{-1}$; 1180 $cm^{-1}$; 760 $cm^{-1}$; 720 $cm^{-1}$.

*Elementary Analysis* (C, H, N) for $C_{18}H_{19}N_3O_5S$

calcd. % C = 51.29; H = 4.54; N = 9.97
found % C = 51.20; H = 4.46; N = 10.01.

The compounds object of the present invention have been characterized from the toxico-pharmacological point of view.

We report the results obtained with the compound 4-pivaloyloxy-3-methyl-2H-1,2-benzothiazine-3-[N-(2-pyridyl)-carboxamide]-1,1-dioxide (CHF 1021) and 4-pivaloyloxy-2-methyl-2H-1,2-benzothiazine-3-[N-(2-thiazolyl)-carboxamide]-1,1-dioxide (CHF 1047).

In these studies the activity of compounds CHF 1021 and CHF 1047 has been compared with the one of piroxicam, one of the most active oxicam previously described, by now entered into the therapeutic practice, and with one of two other esters; valerate and n-butyrate of piroxicam (respectively CHF 1109 and CHF 1115).

Acute toxicity

The toxicity by single administration has been determined by oral route in male mice IVA:NMRI (SPF), fasting, with water ad libitum 18 hours before the experiment. The substances under exam were suspended in 1% carboxymethylcellulose. The results relative to the approximate $LD_{50}$ values, determined by interpolation on Probitpaper, have been reported in Table I.

TABLE I

| Compound | Approx. $LD_{50}$ mg/kg |
|---|---|
| CHF 1021 | 1800 |
| CHF 1047 | >3000 |
| CHF 1109 | 1100 |
| CHF 1115 | 880 |
| Piroxicam | 345 |

Antiinflammatory activity in the test of plantar edema by carrageenin

Crl: CD(SD) male rats, weighing 170—205 g, previously subjected to an acclimation period of at least one week through housing in environment with constant thermohygrometric conditions and fasting with water ad libitum 18 hours before the start of the test, have been distributed "at random" in groups of 5—6 animals each (4 groups corresponding to 4 different dose levels for each compound). A group of control animals has been treated only with the vehicle.

The test has been performed in 2 subsequent experimental sessions:

1: piroxicam versus CHF 1021

2: piroxicam versus CHF 1047, CHF 1109 and CHF 1115.

The activity of the examined compounds, administered at different doses, by the oral route, has been valued by measuring the protection offered against the development of the oedema induced in the rat's paw by injecting (at a distance of one hour from the administration of the active principle) 0.1 ml of 1% carrageenin in physiologic solution in the subplantar aponeurosis of the rear right paw, according to the method of Winter (Winter C. A. et al., Proc. Soc. Exp. Biol. Med. 111, 544, 1962).

The volume of the treated paw has been measured by means of a water-mercury pletismometer according to Leuce P., (Arch. Int. Pharmacodyn. 136, 237, 1962) immediately before and at different times after injection of the phlogogenic agent. The results have been expressed as $ED_{30}$ values calculated on the logarithmic regression straight lines of the percent dose/inhibition of the oedema development, in turn determined from the ratio between the mean AUC value (area under the curve representing the development of the paw volume in the time) in the different treated animals groups and the respective control groups. Such results are shown in the Table II.

Gastrolesive activity

On the same animals on which the antiinflammatory activity has been determined, the gastrolesivity has been contemporaneously checked through macroscopic control of the gastric mucosa at a distance of 7 hours from the administration of the substance under examination.

For each treatment, the logarithmic regression straight lines of the dose — mm of ulceration (single values of each animal) have been determined.

On the ground of such lines the calculation of $UD_3$ values, that is a dose whereby a gastric lesion of the total extension of 3 mm is expected, has been performed.

The results obtained are shown in Figure II.

From the examination of the results, altogether represented in table I and II the following considerations can be found. The compound CHF 1021 endowed with remarkable antiinflammatory activity, comparable to the one of the reference compound (piroxicam), surprisingly exhibits an ulcerogenic activity at least 10 times lower and an acute toxicity about 35% lower in comparison to the reference compound.

These properties of important antiinflammatory activity with considerable decrease of the ulcerogenic effect, constituting the most serious side-effect of non-steroidal antiinflammatory drugs, confer to the product in question a very remarkable therapeutic interest and they turn out so much unexpected if compared with the results obtained with the other analogous derivatives contemporaneously tested.

In fact:

— the compound CHF 1047 or pivalic ester of 4-hydroxy-2-methyl-2H-1,2-benzothiazine-3-[N-(2-thiazolyl)-carboxamide]-1,1-dioxide (sudoxicam) presented a very low antiinflammatory activity. His direct parent compound, sudoxicam, on the contrary, had explicated in analogous tests an antiinflammatory activity comparable to the one of piroxicam, our reference drug (Wiseman E. H., Lombardi no J. G.: Oxicams — A novel family of Non-Steroidal Anti-inflammatory Drugs — Eur. J. Rheumatol. Inflamm. 4(3), 280, 1981).

In this case, therefore, the esterification, at least with this kind of substituent, determined a very high loss of activity.

TABLE II

Data of the comparison tests between compound CHF 1021, CHF 1047, CHF 1109, CHF 1115 and piroxicam. Values of $ED_{30}$ (antiinflammatory activity) and of $UD_3$ (gastrolesive activity) are relative to the oral route and are expressed in µmol/kg

| Exp. session | Compounds | Antiinflammatory activity $ED_{30}$ (µmol/kg) | Gastrolesive activity $UD_3$ (µmol/kg) | Therapeutic index $UD_3/ED_{30}$ | Relative therapeutic index* |
|---|---|---|---|---|---|
| I | piroxicam | 7.8 | 9.3 | 1.2 | 1 |
| | CHF 1021 | 7.9 | 97.2 | 12.3 | 10.25 |
| II | piroxicam | 9.7 | 27.2 | 2.8 | 1 |
| | CHF 1047 | ≃762 | 121.2 | 0.2 | 0.07 |
| | CHF 1109 | 7.0 | 28.2 | 4.0 | 1.43 |
| | CHF 1115 | 5.7 | 23.9 | 4.2 | 1.50 |

* Therapeutic index relative to piroxicam considered as 1.

Compounds CHF 1109 and 1115 although having a good antiinflammatory activity, analogous or also slightly higher than the one of the parent compound, do not show an improvement of tolerability (on the contrary CHF 1115, n-butirryl ester of piroxicam, is slightly more ulcerogenic with a potency ratio of 0.88 to 1), whereby their relative therapeutic index is a little higher than the one of the reference compound: respectively 1.43 and 1.50.

Other pharmacological characteristics of CHF 1021

In other tests of direct comparison between piroxicam and CHF 1021, the latter confirmed its anti-inflammatory activity, along with a good analgesic and antipiretic activity. The potency ratios (ratios between $ED_{50}$ values) calculated on molar basis point out that CHF 1021 is 1.52—0.49—0.72—0.71 times as active as the reference compound on the plantar oedema by nystatin in the rat, on the erythema by U.V. in the guinea pig, on the writhing test by phenylquinone in the mouse and on the fever by beer's yeast in the rat. The two compounds are almost equally active on the arthritis by adjuvant in the rat.

Preliminar clinical data allowed to point out that CHF 1021 confirms to be a valid antiinflammatory drug practically equally active in comparison with piroxicam but with a clearly lower incidence of side effects (about 1/3 than that of the reference drug).

The present invention refers moreover to pharmaceutical compositions containing as the active principle a compound of formula (I), as defined above, as such or as a pharmaceutically acceptable salt, in association with at least one pharmaceutically acceptable excipient.

The compositions can be administered orally, parenterally or topically, in the form respectively of capsules, tablets or similar compositions, suppositories, vials, cream or gel.

For the preparation of the pharmaceutical formulation for the oral administration in unitary dose, the active principle can be mixed with a solid powdered, excipient, such as for instance lactose, saccharose, sorbitol, mannitol, potato or cereal or maize starch, or amylopectin, a derivative of cellulose or gelatin, and it can moreover contain lubricant such as talcum, polyethylenglycol or silica, magnesium or calcium stearate.

The tablets can be variously coated according to well-known methods in the pharmaceutical art. Capsules of hard gelatin can contain granulates of the active principle together with solid powdered excipients, such as lactose, saccharose, sorbitol, mannitol, starches (of the kind indicated above), cellulose or gelatin derivatives, and they may also contain stearic acid or magnesium stearate or talcum.

Unitary doses for rectal administration may be in the form of suppositories containing the active principle in association with a neutral fat base (example glycerides of fat acids) or with water-soluble or autoemulsifiable excipients (example polyethylenglycol mixtures).

For injectable compositions for parenteral administration, the excipients can be a pharmaceutically acceptable sterile liquid such as water or an aqueous solution of polyvinylpyrrolydone or, again, an oil such as, for instance, peanut-oil and, if necessary a stabilizing agent and/or a buffer.

The active principle can be dissolved in the liquid and sterilized on filter before being put in a vial or it can be suitably lyophilized; in this case vials of liquid for injections will be added in the packagings in order to reconstitute the solution before use.

In the case both of the composition in suppositories and in vials a local anaesthetic, if necessary, can be added to the excipients.

The unitary dose for the formulations described above can vary from 10 to 200 mg of active principle and the daily administration will be preferably single.

For the preparation of formulations for topic use, fat-base excipients, such as Vaseline, paraffin oil, lanoline etc, can be used, as well as autoemulsifiable excipients such as alcohols, fats, polyethylenglycols, ethers or esters of fat acids or other tensides emulsified in water in the case of unguents, ointments and creams. On the contrary, in the case of preparations of gel of hydrophylic colloids, polymers of different kinds will be used, such as carboxyvinylpolymers, sodium carboxymethylcellulose, methylcellulose, Methocel gelatinized in water, ethanol, propylenglycol, glycerole, polyethylenglycols, etc.

The topic preparations indicated above can be advantageously added to suitable antibacterial agents, such as parabens, phenol derivatives quaternary ammonium salts, etc.

The concentration of the active principle can vary in these compositions from 1 to 10%.

We report some formulations as exemplification.

Formulation in hard gelatin capsules for oral administrations

Composition at three different dosages:

| | | | | |
|---|---|---|---|---|
| CHF 1021 | mg | 30 | 40 | 60 |
| Microcrystalline cellulose (Avicel ®) | „ | 37.5 | 35 | 30 |
| Dihydrated dibasic calcium phosphate (Encompress ®) | „ | 105 | 98 | 84 |
| Talcum | „ | 6.54 | 6.1 | 5.23 |
| Magnesium stearate | „ | 0.96 | 0.9 | 0.77 |

For the preparation of N. 5000 capsules, with the components that are proportionally present in the above-defined quantities in relation to the dosage of the active principle, the following operations are carried out.

The raw materials are sieved, they are loaded in a powder mixer, the mixture is homogenized. The homogeneous mixture thus obtained is divided into capsules or opercula of hard gelatin by means of operculum-machine.

The weight of unitary mixture for capsule is of 180 mg.

Formulations in suppositories for rectal administration

Composition at three different dosages:

| | | | | |
|---|---|---|---|---|
| CHF 1021 | mg | 30 | 60 | 100 |
| Semi-synthetic glycerides (Witepol ®) | „ | 1570 | 1540 | 1500 |

For the preparation of N. 5000 suppositories, with the components that are proportionally present in

the above-defined quantities in relation to the dosage of the active principle, the following operations are carried out.

The mass of excipient is melted at 40°C. The active principle is incorporated in the melted mass operating with a suitable mechanic dispersant. The mass is cooled at 36°C and it is poured, keeping the suppository mass under stirring, in PVC or aluminium valves having the unitary volume of 1.8 ml. The weight of the suppository mass distributed for each valve is of 1.6 g. It is let solidify and the containers are suitably sealed.

Formulations in cream for topic administration

Compositions at two different dosages of a cream of oil-water emulsion kind:

| | | | |
|---|---|---|---|
| CHF 1021 | g | 2 | 5 |
| Octyldodecanole (Eutanol G®) | " | 7 | 7 |
| Liquid triglyceride $C_8$ (Miritol 218 ®) | " | 3 | 3 |
| Polioxyethylene cetostearyl alcohol (Emulgin $B_1/B_2$ ®) | " | 2 | 2 |
| Propylenic glycole | " | 5 | 5 |
| Carboxyvinylpolymer (Carbopol 940 ®) | " | 1 | 1 |
| Fenocombin ® | " | 1 | 1 |
| Sodium hydroxide Q.S. at pH 5.5 Q.S. at pH 5.5 | | | |
| Purified water Q.S. at g 100 Q.S. at g 100 | | | |

For the preparation of kg 5 of cream, with the components that are proportionally present in the above-defined quantities in relation to the concentration of the active principle, the following operations are carried out.

The carboxyvinylpolymer is dispersed in water (20% of the quantity necessary to the preparation of the lot) and it is neutralized with soda in the quantity required to obtain a pH 5.5. The components and the fat phase are united in a suitable foundry and they are melted at the temperature of 70°C.

The active principle CHF 1021 is dispersed in glycol and water (10% of the quantity necessary to the preparation of the lot), the preservative is dissolved in the residual quantity of water and the solution is taken to 80°C.

The aqueous phase is poured in the fat phase carrying out the homogenization with suitable emulsifier. It is cooled to 40°C and the emulsion is added with the hydroglycolic suspension of the active principle. The emulsion is finally stabilized with the gel of CV Polymer added and dispersed by means of suitable mechanic stirrer.

pH is checked and adjusted at 5.5.

Cream is distributed in flexible aluminium tubes or in other suitable packaging material for preparations for topic use.

**Claims for the Contracting States: DE FR GB IT NL SE**

1. 4-Pivaloyloxy-2-methyl-2H-1,2-benzothiazine-3-[N-(2-pyridyl)-carboxamide]-1,1-dioxide.
2. 4-Pivaloyloxy-2-methyl-2H-1,2-benzothiazine-3-[N-(2-thiazolyl)-carboxamide]-1,1-dioxide.
3. Process for the preparation of the compounds of claims 1 and 2, characterized in that: a N-arylchloro-acetamide (VIII)

$$X—CH_2—CONH—Ar \qquad (VIII)$$

where X means a halogen atom and Ar is 2-pyridyl or 2-thiazolyl is reacted with the intermediate (VII) of formula

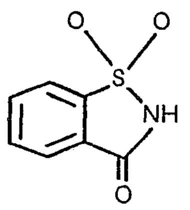

(VII)

to obtain a compound of the general formula (IX)

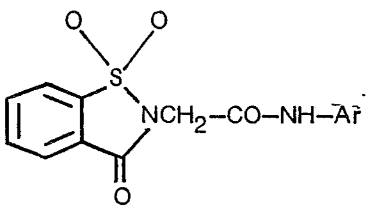

(IX)

where Ar has the above indicated meaning and in a single step compound (IX) turns into the benzothiazinic derivative (X) of the formula:

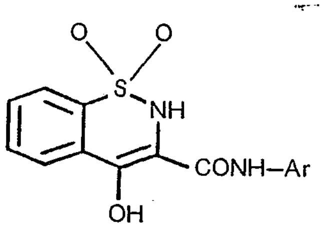

(X)

where Ar has the above indicated meaning and compound (X), as such or in the form of one of its addition salts, is esterified at the hydroxyl group by reaction with a pivaloyl acid derivative and it is finally methylated at the nitrogen atom in 2.

4. Process according to claim 3, characterized in that the transformation of IX in X takes place in the presence of sodium methoxide and dimethylsulfoxide at temperature no higher than 30—35°C.

5. Process according to claim 3, characterized in that the esterification reaction can take place in the absence or in the presence of solvents, bases and catalysts and at temperatures ranging from 0 to 100°C, preferably at room temperature.

6. Pharmaceutical formulation with analgesic and antirheumatic activity, containing as active principle the compound according to claim 1 or 2, or one of its pharmaceutically acceptable salts.

7. Pharmaceutical formulation according to claim 6 for oral, rectal or parenteral administration in the form of capsules, tablets coated if necessary, suppositories or vials, containing from 10 to 200 mg of active principle per unit dose.

8. Pharmaceutical composition according to claim 6, for topic administration, in the form of cream or gel, containing the active principle in concentration from 1 to 10%.

**Claims for the Contracting State: AT**

1. Process for the preparation of compounds of general formula (I)

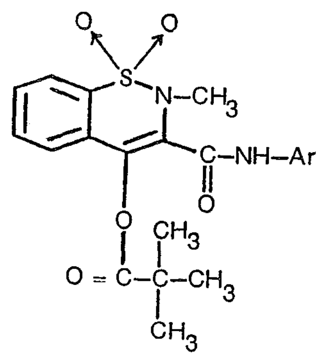

(I)

where Ar stands for 2-pyridyl-, 2-thiazolyl- residues, characterized in that: a N-arylchloroacetamide (VIII)

$$X—CH_2—CONH—Ar \qquad (VIII)$$

where X means a halogen atom and Ar has the above indicated meaning, is reacted with the intermediate (VII) of formula

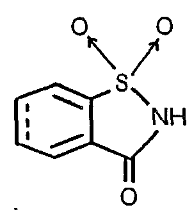

(VII)

to obtain a compound of the general formula (IX)

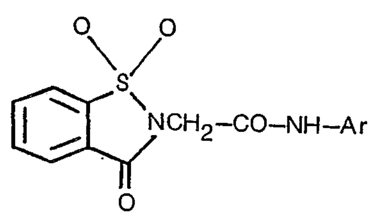

(IX)

where Ar has the above indicated meaning and in a single step compound (IX) turns into the benzothiazinic derivative (X) of formula:

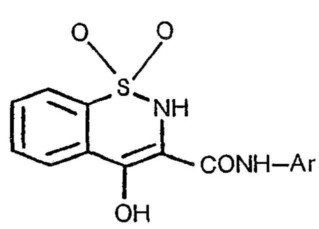

(X)

where Ar has the above indicated meaning, and compound (X), as such or in the form of one of its addition salts, is esterified at the hydroxyl group by reaction with a pivaloyl acid derivative it is finally methylated at the nitrogen atom in 2.

2. Process according to claim 1, characterized in that the transformation of IX in X takes place in the presence of sodium methoxide and dimethylsulfoxide at temperature no higher than 30—35°C.

3. Process according to claim 1, characterized in that the esterification reaction can take place in the absence or in the presence of solvents, bases and catalysts and at temperatures ranging from 0 to 100°C, preferably at room temperature.

**Patentansprüche für die Vertragsstaaten: DE FR GB IT NL SE**

1. 4-Pivaloyloxy-2-methyl-2H-1,2-benzothiazin-3-[N-(2-pyridyl)-carboxamid]-1,1-dioxid.

2. 4-Pivaloyloxy-2-methyl-2H-1,2-benzothiazin-3-[N-(2-thiazolyl)-carboxamid]-1,1-dioxid.

3. Verfahren zur Herstellung der Verbindungen der Ansprüche 1 und 2, dadurch gekennzeichnet, daß man ein N-Arylchloracetamid (VIII)

$$X—CH_2—CONH—Ar \qquad (VIII)$$

worin X ein Halogenatom bedeutet und Ar 2-Pyridyl oder 2-Thiazolyl ist, mit dem Zwischenprodukt (VII) der Formel

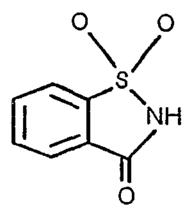

(VII)

umsetzt, um eine Verbindung der allgemeinen Formel (IX)

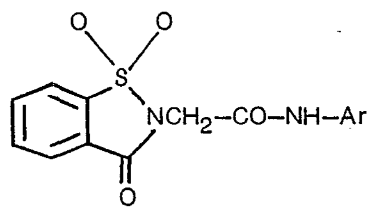

(IX)

zu erhalten, worin Ar die oben angegebene Bedeutung hat, und daß man in einer einzigen Stufe die Verbindung (IX) in das Benzothiazinderivat (X) der Formel

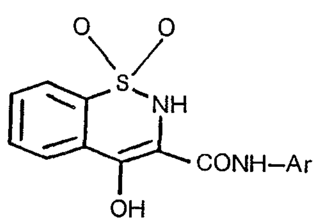

(X)

worin Ar die oben angegebene Bedeutung hat, umwandelt und die Verbindung (X) als solche oder in Form eines ihrer Säureadditionssalze an der Hydroxylgruppe durch Reaktion mit einem Pivaloylsäurederivat verestert und am Schluß am Stickstoffatom in 2 methyliert.

4. Verfahren nach Anspruch 3, dadurch gekennzeichnet, daß die Umwandlung von IX in X in Gegenwart von Natriummethoxid und Dimethylsulfoxid bei einer Temperatur erfolgt, die nicht höher als 30 bis 35°C ist.

5. Verfahren nach Anspruch 3, dadurch gekennzeichnet, daß die Veresterungsreaktion in Abwesenheit oder in Gegenwart von Lösungsmitteln, Basen und Katalysatoren und Temperaturen im Bereich von 0 bis 100°C, vorzugsweise bei Raumtemperatur, stattfinden kann.

6. Arzneimittel mit analgetischer und antirheumatischer Aktivität, dadurch gekennzeichnet, daß es als Wirkstoff die Verbindung nach Anspruch 1 oder 2 oder eines ihrer pharmazeutisch annehmbaren Salze enthält.

7. Arzneimittel nach Anspruch 6 zur oralen, rektalen oder parenteralen Verabreichung in Form von Kapseln, Tabletten, die erforderlichenfalls beschichtet sind, Suppositorien oder Ampullen, die 10 bis 200 mg Wirkstoff pro Einheitsdosis enthalten.

8. Arzneimittel nach Anspruch 6 zur topischen Verabreichung in Form einer Creme oder eines Gels, welche bzw. welches den Wirkstoff in einer Konzentration von 1 bis 10% enthält.

**Patentansprüche für den Vertragsstaat: AT**

1. Verfahren zur Herstellung von Verbindungen der allgemeinen Formel (I)

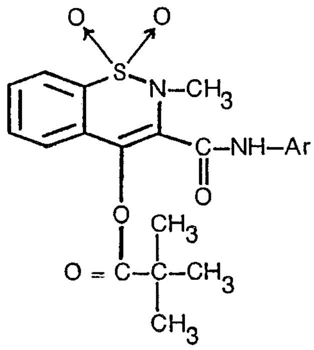

(I)

worin Ar für 2-Pyridyl-, 2-Thiazolylreste steht, dadurch gekennzeichnet, daß man ein N-Arylchloracetamid (VIII)

$$X—CH_2—CONH—Ar \qquad (VIII)$$

worin X für ein Halogenatom steht und Ar die oben angegebene Bedeutung hat, mit dem Zwischenprodukt (VII) der Formel

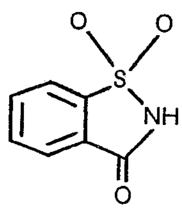

(VII)

umsetzt, um eine Verbindung der allgemeinen Formel (IX)

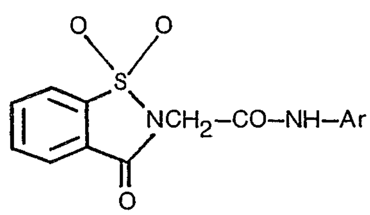

(IX)

zu erhalten, worin Ar die oben angegebene Bedeutung hat, und daß man in einer einzigen Stufe die Verbindung (IX) in das Benzothiazinderivat (X) der Formel

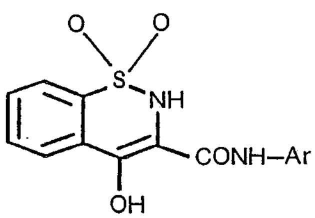

(X)

worin Ar die oben angegebene Bedeutung hat, umwandelt und die Verbindung (X) als solche oder in Form eines ihrer Säureadditionssalze an der Hydroxylgruppe durch Reaktion mit einem Pivaloylsäurederivat verestert und am Schluß am Stickstoffatom in 2 methyliert.

2. Verfahren nach Anspruch 1, dadurch gekennzeichnet, daß die Umwandlung in IX in X in Gegenwart von Natriummethoxid und Dimethylsulfoxid bei einer Temperatur erfolgt, die nicht höher als 30 bis 35°C ist.

3. Verfahren nach Anspruch 1, dadurch gekennzeichnet, daß die Veresterungsreaktion in Abwesenheit oder in Gegenwart von Lösungsmitteln, Basen und Katalysatoren und Temperaturen im Bereich von 0 bis 100°C, vorzugsweise bei Raumtemperatur, stattfinden kann.

**Revendications pour les Etats contractants: DE FR GB IT NL SE**

1. Pivaloyloxy-4-méthyl-2-benzothiazine-2H-1,2-[N-(pyridyl-2-)-carboxamide]-3-dioxyde-1,1.
2. Pivaloyloxy-4-méthyl-2-benzothiazine-2H-1,2-[N-(thiazolyl-2)-carboxamide]-3-dioxyde-1,1.
3. Procédé pour la préparation des composés des revendications 1 et 2, caractérisé en ce que:
— on fait réagir un N-arylchloroacétamide (VIII)

$$X—CH_2—CONH—Ar \qquad (VIII)$$

où X représente un atome d'halogène et Ar est un pyridyl-2 ou un thiazolyl-2, avec le composé intermédiaire (VII) de formule

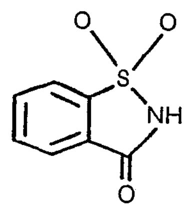

(VII)

pour obtenir un composé de formule générale (IX)

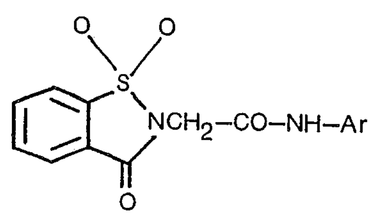

(IX)

dans laquelle Ar présente la signification indiquée ci-dessus et, en une seule étape, le composé (IX) se transforme en dérivé benzothiazinique (X) de formule

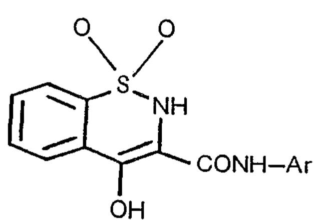

(X)

dans laquelle Ar présente la signification indiquée ci-dessus et,
— on estérifie sur le groupe hydroxyle le composé (X) tel quel ou sous forme de l'un de ses sels d'addition avec un dérivé d'acide pivaloïque, et finalement
— on méthyle sur l'atome d'azote en 2.

4. Procédé selon la revendication 3, caractérisé en ce que la transformation de IX et X s'effectue en présence de méthoxyde de sodium et de diméthylsulfoxyde à une température ne dépassant pas 30—35°C.

5. Procédé selon la revendication 3, caractérisé en ce que la réaction d'estérification peut s'effectuer en l'absence ou en présence de solvants, de bases et de catalyseurs et à des températures de l'ordre de 0 a 100°C, de préférence à température ambiante.

6. Formulation pharmaceutique ayant une activité analgésique et antirhumatismale, contenant comme principle actif le composé selon la revendication 1 ou 2 ou l'un de ses sels pharmaceutiquement acceptables.

7. Formulation pharmaceutique selon la revendication 6, pour l'administration orale, rectale ou parentérale, sous forme de capsules, de comprimés avec revêtement si nécessaire, suppositoires ou fiales, contenant de 10 à 200 mg de principe actif par dose unitaire.

8. Composition pharmaceutique selon la revendication 6, pour administration topique, sous forme de crème ou de gel contenant le principe actif en une concentration de 1 a 10%.

**Revendications pour l'Etat contractant: AT**

1. Procédé pour la préparation de composés de formule générale I:

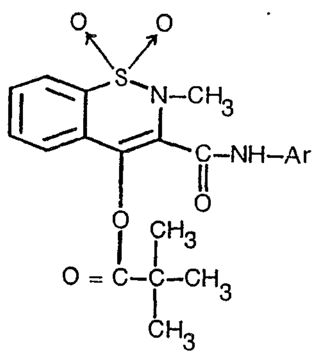

(I)

dans laquelle Ar représents des restes pyridyl-2, thiazolyl-2, caractérisé en ce que:

— on fait réagir un N-arylchloroacétamide (VIII)

$$X—CH_2—CONH—Ar \qquad (VIII)$$

où X représente un atome d'halogène et Ar présente le signification donnée ci-dessus avec le composé intermédiaire (VII) de formule:

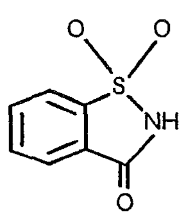

(VII)

pour obtenir un composé de formule générale (IX)

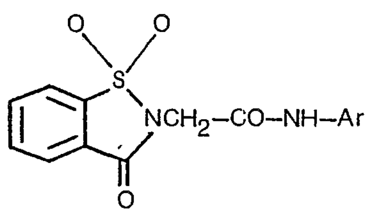

(IX)

dans laquelle Ar présente la signification indiquées ci-dessus et, en une seule étape, le composé (IX) se transforme en dérivé benzothiazinique (X) de formule:

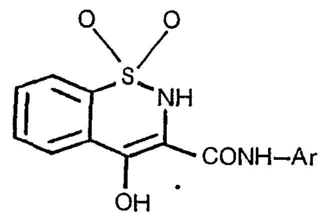

(X)

dans laquelle Ar présente la signification indiquée ci-dessus et,

— on estérifie sur le groupe hydroxyle le composé (X), tel quel ou sous forme de l'un de ses sels d'addition avec un dérivé d'acide pivaloïque, et finalement

— on méthyle sur l'atome d'azote en 2.

2. Procédé selon la revendication 1, caractérisé en ce que la transformation de IX et X s'effectue en présence de méthoxyde de sodium et de diméthylsulfoxyde à une température ne dépassant pas 30—35°C.

3. Procédé selon la revendication 3, caractérisé en ce que la réaction d'estérification peut s'effectuer en l'absence ou en présence de solvants, de bases et de catalyseurs et à des températures de l'ordre de 0 à 100°C, de préférence à une température ambiante.